# EUROPEAN PATENT SPECIFICATION

(11) **EP 3 123 190 B1**
(45) Date of publication and mention of the grant of the patent: **28.02.2018**
(21) Application number: 15717963.1
(22) Date of filing: 17.03.2015
(51) Int. Cl.: G01R 33/34, G01R 33/341, A61F 5/058

(54) **VACUUM SPLINT WITH RADIO FREQUENCY COIL FOR MAGNETIC RESONANCE IMAGING**
VAKUUMSPLINT MIT RADIOFREQUENZSPULE FÜR MAGNETRESONANZBILDGEBUNG
ATTELLE SOUS VIDE COMPRENANT UNE BOBINE DE RADIO FRÉQUENCE POUR L'IMAGERIE PAR RÉSONANCE MAGNÉTIQUE

(30) Priority: 27.03.2014 US 201461970968 P
(43) Date of publication of application: 01.02.2017
(73) Proprietor: Koninklijke Philips N.V., 5656 AE Eindhoven (NL)
(72) Inventor: REESE, David Gregory, NL-5656 AE Eindhoven (NL)
(74) Representative: van Iersel, Hannie
(86) International application number: PCT/IB2015/051922
(87) International publication number: WO 2015/145298

(56) References cited:
- WO-A2-2013/012717
- US-A1- 2007 233 226
- US-A1- 2012 277 644
- US-A1- 2012 293 174
- US-A1- 2013 193 610

## Description

### Background

Vacuum splints are commonly used in emergency situations as temporary splints to immobilize an injured body part. Magnetic resonance imaging is a common tool used to diagnose a wide variety of injuries that patients may suffer. Performance of magnetic resonance imaging requires the use of receive coils proximate to the injured anatomy, which may be made difficult by the presence of a vacuum splint or other type of splint stabilizing the injured anatomy.

From US 2012/277644 A1 a device for supporting a body part according to the preamble of appended independent claim 1, namely a vacuum splint for maintaining a person in an immobilized state, is known. The vacuum splint includes a flexible airtight casing upon which a patient is placed. The casing has an internal chamber that contains a large number of beads. Valves are provided to develop a vacuum within the chamber to convert the casing from a relatively soft, flexible state to a rigid, evacuated state. A semi-rigid member spans the length of the vacuum splint that resists longitudinal compression when air is evacuated from the chamber. The semi-rigid member also retains sufficient longitudinal and lateral flexibility such that it allows the vacuum splint to be molded to various patient positions and folded or rolled up when not in an evacuated state. The head end of the vacuum splint includes two protrusions that extend upward. When in an evacuated state, these protrusions extend over either side of a patient's head thereby providing support and protection.

In US 2012/293174 A1 a Radio Frequency (RF) coil apparatus for generating a Magnetic Resonance (MR) image includes a body adapted to be worn by a subject being scanned, the body comprising an anterior portion, a posterior portion, and a transition portion coupled between the anterior and posterior portions, a first RF receive-only saddle coil including a first coil positioned in the anterior portion and a second coil positioned in the anterior portion, the first RF saddle coil configured to be positioned on the anterior and posterior sides of the subject.

In US 2007/233226 A1 tissue stabilization and ablation devices and methods are described. These devices and methods provide techniques for stabilizing and ablating body tissues during surgical ablation procedures. In many embodiments, for example, devices may be used in minimally invasive techniques for ablating epicardial tissue adjacent one or more pulmonary veins to treat atrial fibrillation. Tissue stabilization and ablation devices generally include a rigidifying bladder coupled with an ablation member. The devices may additionally include a tissue stabilizing bladder or means within the rigidifying bladder for enhancing tissue stabilization. The rigidifying bladder conforms to a tissue surface and then stiffens to help the device hold its shape and position and to stabilize the tissue. The ablation member is then used to ablate an area of tissue. Such cardiac stabilization and ablation devices and methods may be used to ablate one or more patterns on the epicardial surface of a heart to treat atrial fibrillation and/or other cardiac arrhythmias.

From US 2013/193610 A1 a method for producing a moulding is known. The shape of the moulding is adapted to an initial mould. According to the method a plurality of layers are deformably placed on top of one another such that said layers can be joined to each other by heat, the layers are deformed by being pressed onto the initial mould and in order to join the layers, heat is applied in the deformed state, wherein in the arrangement made of layers, at least one converter element is introduced that transforms supplied energy into heat energy and with which at least parts of the layers are heated to join the layers.

Finally, WO 2013/012717 A2 describes an orthopaedic fixation device that includes an inner core and shaft formed of a multi-layered, fiber-reinforced composite. A sensing element is embedded within the multi-layered, fiber-reinforced composite.

### Summary of the Invention

The invention is described in the independent claims. Preferred embodiments are described in the sub claims.

In accordance with the present invention there is provided a device for supporting a body part as defined in independent claim 1. The device includes a sleeve adapted to support the body part. The sleeve contains a chamber that is configured to have substantially all of the air contained therein evacuated. The device also includes a flexible radiofrequency surface receive coil disposed within the chamber.

A device according to the present invention may be used in a method as defined in independent claim 11. This method includes securing a sleeve about an injured body part. The sleeve contains a chamber that is configured to have substantially all of the air contained therein evacuated. A flexible radiofrequency surface coil is disposed within the chamber. The method further includes evacuating substantially all of the air from the chamber. The method further includes coupling the flexible radiofrequency surface coil to a magnetic resonance imaging device. The method further includes imaging the injured body part using the magnetic resonance imaging device. The flexible radiofrequency surface coil operates as a receive coil for the imaging.

### Brief Description Of The Drawings

Figure 1 illustrates a prior art vacuum splint.
Figure 2A illustrates a first view of a prior art device for applying a radiofrequency surface receive coil to a patient.
Figure 2B illustrates a second view of the device of Figure 2A.
Figure 3 illustrates a vacuum splint with integrated radiofrequency surface receive coil according to an exemplary embodiment.
Figure 4 illustrates an exemplary method for use of a vacuum splint with integrated radiofrequency surface receive coil such as the exemplary vacuum splint of Figure 3.
Figure 5 illustrates a vacuum splint with integrated radiofrequency surface receive coil according to a further exemplary embodiment.

### Detailed Description

The exemplary embodiments may be further understood with reference to the following description and the related appended drawings, wherein like elements are provided with the same reference numerals. Specifically, the exemplary embodiments relate to vacuum splints with integrated radiofrequency surface receive coils for providing immobilization and supporting magnetic resonance imaging.

Practitioners of emergency medicine commonly use vacuum splints to temporarily immobilize an injured body part. Vacuum splints are commonly used to immobilize trauma-related injuries such as joint dislocations, subluxations, and extremity fractures. Vacuum splints typically comprise an outer vinyl sheeting layer that is filled with a large quantity of polystyrene balls on the order of 2 millimeters in diameter. When air is evacuated from the interior of the sleeve, the splint becomes rigid, immobilizing the injured area.

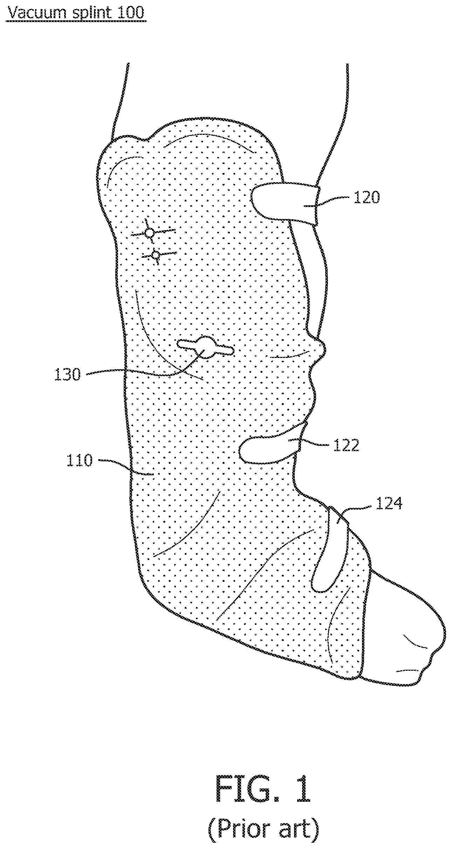

Because a vacuum splint shrinks to conform to any shape, it may be used on any size patient; its quick and easy application makes it particularly effective for splinting of fractures in children. Further, because a vacuum splint contains no obstructive metal components, it is radiolucent and does not interfere with the radiographic image of the injured limb. Figure 1 illustrates a prior art vacuum splint 100. The vacuum splint 100 may comprise a layer of outer sheeting 110 forming a sleeve that is adapted to surround an area of a patient's anatomy. The outer sheeting 110 of the vacuum splint has a chamber therein that is filled with 2-millimeter expanded polystyrene balls (not shown). The vacuum splint 100 is secured about the patient's anatomy by means of straps 120, 122 and 124 that secure with hook-and-loop fasteners. Air may be evacuated from the inner chamber via valve 130. Further background on vacuum splints may be found in "The Vacuum Splint: An Aid In Emergency Splinting of Fractures" by Letts et al., CMA Journal, Oct. 6, 1973, Vol. 109, pp. 599-600.

Magnetic resonance imaging ("MRI") devices are commonly used to diagnose a wide variety of conditions in a patient. Among the components of an MRI device are radiofrequency ("RF") transmitting coils generating electromagnetic fields that are transmitted into the patient, and RF receiving coils detecting resulting electromagnetic radiation from the patient. The detected radiation is then analyzed to yield an image that medical professionals may inspect to make a diagnosis for the patient.

RF coils may fall into two general categories: volume coils and surface coils. Surface coils are typically single-turn or multi-turn loops that are placed directly over the patient's anatomy of interest. Surface coils are sensitive to signals that are close to the coil, and therefore are often used as RF receiver coils because they detect the signal in an organ of interest more efficiently. Although surface coils amplify noise as efficiently as they amplify signal, noise from the rest of the patient is reduced due to the localized nature of the surface coils. Further, although surface coils are placed on or around the surface of a patient, they may be optimized to image deep-body structures. However, surface coils are typically contained within rigid bodies that may not always provide the best fit for all patients.

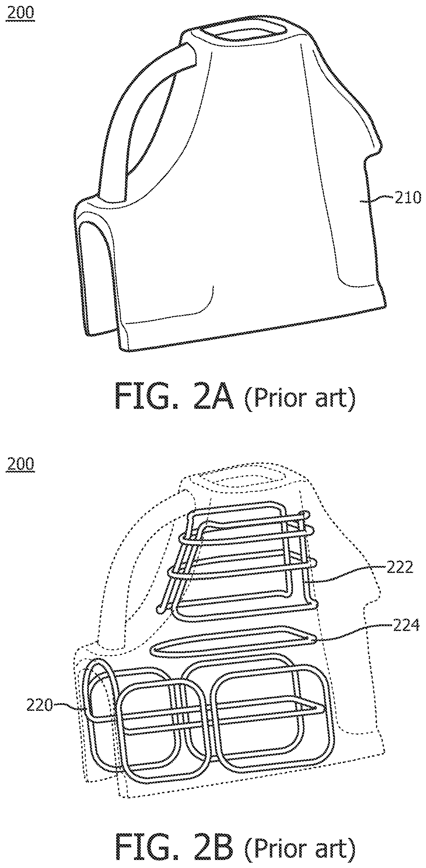

Figures 2A and 2B illustrate a prior art RF coil 200 with integrated RF receive elements. The RF coil 200 shown in Figures 2A and 2B is adapted to be applied to a patient's foot and ankle, but it will be apparent to those of skill in the art that different types of RF coil may be adapted to be applied to different body parts. Figure 2A illustrates the rigid outer shell 210 of the RF coil 200, which may be, for example, made from a rigid plastic or any other appropriate non-conductive material. Figure 2B illustrates the same RF coil 200 with the outer shell 210 rendered transparently. Figure 2B also illustrates receive elements 220, 222 and 224 that may serve to detect radiation to enable medical imaging as described above. It will be further apparent to those of skill in the art that the quantity, shape and arrangement of the receive elements 220, 222 and 224 is only exemplary and that these aspects may vary for RF coils adapted to be applied to different body parts or differing versions of an RF coil adapted to be applied to the same body part.

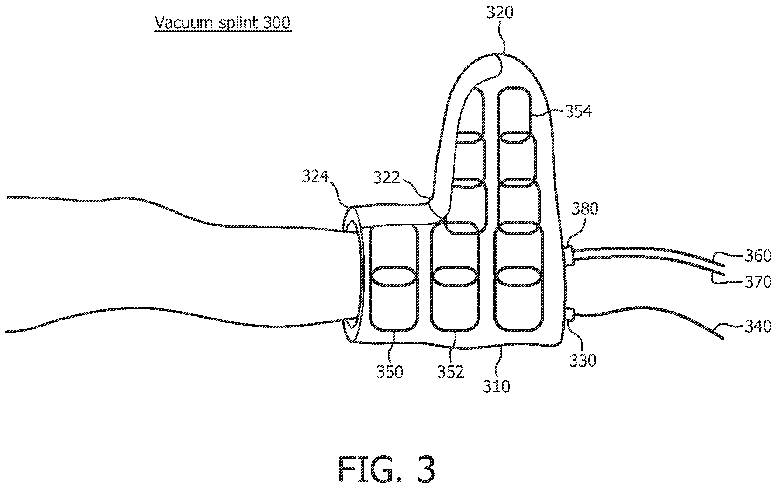

In accordance with the present invention, the exemplary embodiments described herein integrate RF receive coils into a vacuum splint. Figure 3 illustrates a vacuum splint 300 according to a first embodiment of the present invention. Like the prior art vacuum splint 100, the exemplary vacuum splint 300 may include outer sheeting 310 that may be vinyl or another appropriate material. The outer sheeting 310 may form a sleeve that is sized and shaped to fit over any part of a patient's anatomy that it may be desirable to immobilize; Figure 3 illustrates a vacuum splint 300 adapted to immobilize a patient's foot and ankle, but those of skill in the art will understand that this is only exemplary. In alternative embodiments, the vacuum splint 300 may be adapted to immobilize a wrist, an arm or portion thereof, a shoulder, an ankle, a leg, a hip, a pelvis, a torso, a neck, or any other desired portion of a patient's anatomy.

The outer sheeting 310 of the vacuum splint 300 may have a chamber therein that is filled with 2-millimeter expanded polystyrene balls. It will be apparent to those of skill in the art that the precise size and material of the balls may vary among differing embodiments, and that other types of particles may be used so long as they are capable of performing as will be described hereinafter. The vacuum splint 300 may also include straps 320, 322 and 324 to secure the vacuum splint 300 around the area to be immobilized. In one exemplary embodiment, the straps 320, 322 and 324 may close by means of hook-and-loop fasteners, but alternate embodiments may use any different type of strap and fastener sufficient to secure the vacuum splint 300 around the immobilized area, while remaining non-metallic in order not to interfere with imaging of the immobilized area. It will be further apparent to those of skill in the art that the precise number and placement of straps to be used may vary in differing embodiments; for example, a splint adapted to immobilize a larger area of the patient's body may include more straps.

The vacuum splint 300 may also include a valve 330 passing through the outer sheeting 310. The valve 330 may be coupled to a vacuum tube 340 or, as an alternative, any other suction allowing air to be evacuated from within the outer sheeting 310. Preferably, a compressor or other driver generating the suction through the vacuum tube 340 to evacuate the air from the vacuum splint 300 may be located at a sufficiently large distance away from any MRI equipment in order not to interfere with the magnetic fields generated to record patient images.

The vacuum splint 300 also includes flexible RF surface receive coil elements 350, 352 and 354 disposed within the outer sheeting 310. The flexible RF surface receive coil elements 350, 352 and 354 may be fabricated from any appropriate metallic material that is capable of flexing as the vacuum splint 300 is opened or closed and capable of functioning as an RF surface receive coil. The coil elements 350, 352 and 354 may typically be made from copper but may alternately be made from any other metallic or non-metallic conductive material capable of performing as described herein. The exemplary embodiments are discussed with reference to a vacuum splint 300 using three flexible RF surface receive coil elements 350, 352 and 354, but in other embodiments, a single flexible RF surface receive coil or varying numbers of elements may be used. The flexible RF surface receive coil elements 350, 352 and 354 may be connected to an MRI device by a coil power source 360 and a transmission cable 370 through a port 380 traversing the outer sheeting 310 in a manner that allows the outer sheeting 310 to remain airtight when the vacuum splint 300 is in use.

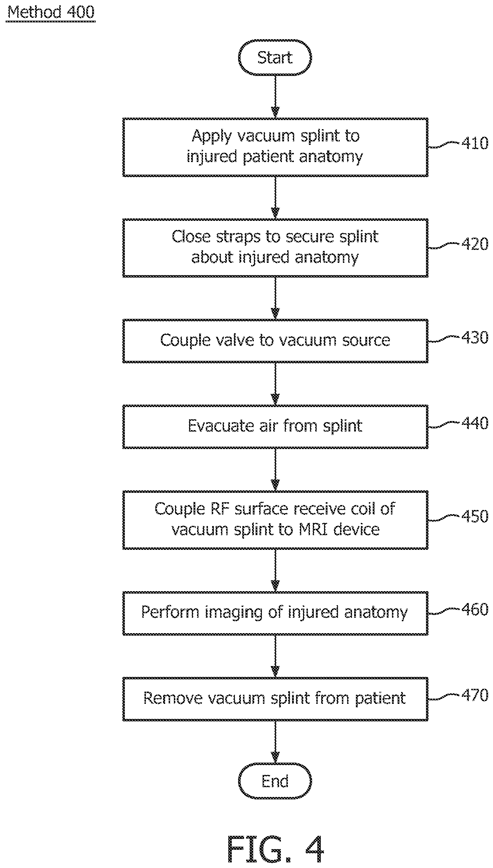

Figure 4 illustrates an exemplary method 400 for using the vacuum splint 300 to immobilize an area of interest on a patient's body and subsequently image the area using an MRI device. The method 400 will be described with reference to performance by a single individual, but it will be apparent that different medical professionals or other appropriate individuals may perform different steps of the method 400. In step 410, a paramedic, other medical professional, or other relevant individual may apply the vacuum splint 300 to the relevant area of the patient's body. In step 420, the individual applying the splint may secure the splint about the injured area by closing straps 330, 332 and 334.

In step 430, the individual may couple the valve 340 to a vacuum source. As noted above, this may be, for example, a vacuum port integrated into an MRI device or nearby; any necessary compressors or vacuum pumps may be located at a safe distance from the MRI device in order not to interfere with the imaging process, with only vacuum tubing near the MRI device itself. In step 440, the vacuum source evacuates air from the inside of the outer sheeting 310. This causes the vacuum splint 300 to contract around the patient's anatomy and become rigid, providing stability and immobilization of the area of interest. The fit provided to the patient may be better than other types of splints, while the surface of the outer sheeting 310 may become slightly irregular, allowing for air circulation to the skin. The flexible RF surface receive coil 350 may also be compressed around the patient's anatomy, creating a rigid coil that is also customized to the patient's anatomy.

In step 450, the flexible RF surface receive coil 350 is coupled to an MRI device through port 360. Because the flexible RF surface receive coil 350 is already in close proximity to the patient's anatomy, no external receive coil need be used in order for imaging to occur. In step 460, MRI imaging of the patient's anatomy may proceed using the MRI device as is known in the art. Once imaging is complete, the vacuum splint 300 may continue to provide stability to the injured anatomy in the manner known in the art. Subsequently, in step 470, the vacuum splint 300 may be removed from the patient's anatomy at an appropriate time; it will be apparent to those of skill in the art that this may be immediately after MRI imaging, after a diagnosis has been made, or at any other appropriate time based on the nature of the patient's condition. After step 470, the method terminates.

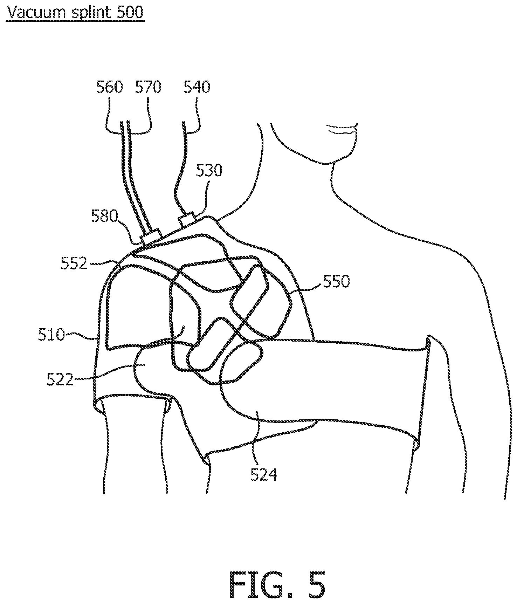

Figure 5 illustrates a vacuum splint 500 according to a further exemplary embodiment of the present invention. The exemplary vacuum splint 500, adapted to be applied to the shoulder of a patient, may include many of the same elements as the exemplary vacuum splint 300 discussed above. Thus, the vacuum splint 500 includes outer sheeting 510 that may be filled with polystyrene balls as described above. The vacuum splint 500 may be adapted to be secured about the area of interest (e.g., the shoulder) by straps 520 and 522 that may be secured, for example, by hook-and-loop closures. The vacuum splint 500 may also include a valve 530 passing through the outer sheeting 510, which may be coupled to a vacuum tube 540 or, as an alternative, any other suction allowing air to be evacuated from within the outer sheeting 510.

The vacuum splint 500 also includes flexible RF surface receive coil elements 550 and 552 disposed within the outer sheeting 510, in the same manner as described above with reference to coil elements 350, 352 and 354 disposed within the vacuum splint 300; the vacuum splint 500 may also include one or more posterior coil elements that are not visible in Figure 5 behind the patient. As was the case for the vacuum splint 300, the precise number and arrangement of the coil elements within vacuum splint 500 is only exemplary, and it will be apparent to those of skill in the art that these aspects may vary among differing embodiments. The flexible RF surface receive coil elements 550 and 552 may be connected to an MRI device by a coil power source 560 and a transmission cable 570 through a port 580 traversing the outer sheeting 510 in a manner that allows the outer sheeting 510 to remain airtight when the vacuum splint 500 is in use. The manner of use for the vacuum splint 500 may be substantially similar to the method 400 described above with reference to the vacuum splint 300.

The exemplary embodiments may provide a patient with substantially equivalent stability and immobilization to those provided by a prior art vacuum splint. Because the coil contained within the exemplary vacuum splint may become rigid once air is vacuumed out of the splint, it may help to limit patient motion. Additionally, because the coil may conform to the patient's anatomy once air is vacuumed out of the splint, it may be more comfortable to the patient than previously existing apparatuses for applying an RF surface receive coil to the patient. As a further benefit of the close conformity to the patient's anatomy of the RF surface receive coil of the exemplary embodiments, better signal-noise ratio may be achieved due to a precise fit around the patient's anatomy. Further, the high degree of stability provided by the exemplary vacuum splint may aid in minimizing motion artifacts in any resulting MRI scans.

It will be apparent to those skilled in the art that various modifications may be made to the exemplary embodiments, without departing from the scope of the invention. Thus, it is intended that the present invention cover modifications and variations of this invention provided they come within the scope of the appended claims.

## Claims

1. A device (300, 500) for supporting a body part, comprising:
a sleeve (310, 510) adapted to support the body part, the sleeve containing a chamber that is configured to have substantially all of the air contained therein evacuated **characterized by**
a flexible radiofrequency surface receive coil (350, 550) for magnetic resonance imaging disposed within the chamber.

2. The device of claim 1, wherein the body part is one of an arm, a wrist, a shoulder, a leg, an ankle, a hip, a pelvis, a torso and a neck.

3. The device of claim 1, further comprising:
a plurality of particles disposed within the chamber, wherein the particles include balls.

4. The device of claim 3, wherein the balls have a diameter substantially equal to two millimeters.

5. The device of claim 3, wherein the balls comprise polystyrene.

6. The device of claim 1, further comprising:
a radiolucent strap (330, 332, 334, 522, 524) for securing the sleeve about the body part.

7. The device of claim 6, wherein the radiolucent strap secures via hook and loop fasteners.

8. The device of claim 1, further comprising:
a port (380, 580) for connecting the flexible radiofrequency surface receive coil (350, 550) to a magnetic resonance imaging device.

9. The device of claim 1, wherein the flexible radiofrequency surface receive coil (350, 550) comprises copper.

10. The device of claim 1, wherein the flexible radiofrequency surface receive coil (350, 550) comprises a plurality of coil elements (350, 352, 354, 550, 552).

11. A method, comprising:
securing a sleeve (310, 510) about an injured body part, the sleeve (310, 510) containing a chamber that is configured to have substantially all of the air contained therein evacuated, a flexible radiofrequency surface coil (350, 550) for magnetic resonance imaging being disposed within the chamber;
evacuating substantially all of the air from the chamber;
coupling the flexible radiofrequency surface coil (350, 550) to a magnetic resonance imaging device; and
imaging the injured body part using the magnetic resonance imaging device, the flexible radiofrequency surface coil (350, 550) operating as a receive coil for the imaging.

12. The method of claim 11, wherein the injured body part is one of an arm, a wrist, a shoulder, a leg, an ankle, a hip, a pelvis, a torso and a neck.

13. The method of claim 11, wherein the sleeve is secured about the injured body part with a radiolucent strap (330, 332, 334, 522, 524).

14. The method of claim 11, further including coupling the flexible radiofrequency surface coil (350, 550) to a magnetic resonance imaging device by way of a port traversing the sleeve (310, 510).

15. The method of claim 11, wherein the flexible radiofrequency surface receive coil (350, 550) comprises a plurality of coil elements (350, 352, 354, 550, 552).

## Patentansprüche

1. Vorrichtung (300, 500) zum Stützen eines Körperteils, umfassend:
eine Manschette (310, 510), die dafür ausgelegt ist, den Körperteil zu stützen, wobei die Manschette eine Kammer enthält, die dafür konfiguriert ist, dass im Wesentlichen die gesamte darin enthaltene Luft evakuiert wird, **gekennzeichnet durch**
eine flexible Hochfrequenz-Oberflächenempfangsspule (350, 550) zur Magnetresonanzbildgebung, die in der Kammer angeordnet ist.

2. Vorrichtung nach Anspruch 1, wobei der Körperteil ein Arm, ein Handgelenk, eine Schulter, ein Bein, ein Fußgelenk, eine Hüfte, ein Becken, ein Rumpf oder ein Hals ist.

3. Vorrichtung nach Anspruch 1, weiterhin umfassend:
eine Vielzahl von Teilchen, die in der Kammer angeordnet sind, wobei die Teilchen Kugeln umfassen.

4. Vorrichtung nach Anspruch 3, wobei die Kugeln einen Durchmesser von im Wesentlichen gleich zwei Millimetern haben.

5. Vorrichtung nach Anspruch 3, wobei die Kugeln Polystyrol umfassen.

6. Vorrichtung nach Anspruch 1, weiterhin umfassend:
einen strahlendurchlässigen Gurt (330, 332, 334, 522, 524) zum Befestigen der Manschette um den Körperteil herum.

7. Vorrichtung nach Anspruch 6, wobei der strahlendurchlässige Gurt mit einem Klettverschluss befestigt wird.

8. Vorrichtung nach Anspruch 1, weiterhin umfassend:
einen Anschluss (380, 580) zum Verbinden der flexiblen Hochfrequenz-Oberflächenempfangsspule (350, 550) mit einer Magnetresonanzbildgebungsvorrichtung.

9. Vorrichtung nach Anspruch 1, wobei die flexible Hochfrequenz-Oberflächenempfangsspule (350, 550) Kupfer umfasst.

10. Vorrichtung nach Anspruch 1, wobei die flexible Hochfrequenz-Oberflächenempfangsspule (350, 550) eine Vielzahl von Spulenelementen (350, 352, 354, 550, 552) umfasst.

11. Verfahren, umfassend:
Befestigen einer Manschette (310, 510) um einen verletzten Körperteil, wobei die Manschette (310, 510) eine Kammer enthält, die dafür konfiguriert ist, dass im Wesentlichen die gesamte darin enthaltene Luft evakuiert wird, wobei eine flexible Hochfrequenz-Oberflächenempfangsspule (350, 550) zur Magnetresonanzbildgebung in der Kammer angeordnet ist;
Evakuieren von im Wesentlichen der gesamten Luft aus der Kammer;
Koppeln der flexiblen Hochfrequenz-Oberflächenspule (350, 550) mit einer Magnetresonanzbildgebungsvorrichtung; und
Abbilden des verletzten Körperteils mithilfe der Magnetresonanzbildgebungsvorrichtung, wobei die flexible Hochfrequenz-Oberflächenspule (350, 550) als eine Empfangsspule für die Bildgebung funktioniert.

12. Verfahren nach Anspruch 11, wobei der verletzte Körperteil ein Arm, ein Handgelenk, eine Schulter, ein Bein, ein Fußgelenk, eine Hüfte, ein Becken, ein Rumpf oder ein Hals ist.

13. Verfahren nach Anspruch 11, wobei die Manschette mit einem strahlendurchlässigen Gurt (330, 332, 334, 522, 524) um den Körperteil herum befestigt wird.

14. Verfahren nach Anspruch 11, weiterhin umfassend das Koppeln der flexiblen Hochfrequenz-Oberflächenspule (350, 550) mit einer Magnetresonanzbildgebungsvorrichtung mithilfe eines die Manschette (310, 510) durchquerenden Anschlusses.

15. Verfahren nach Anspruch 11, wobei die flexible Hochfrequenz-Oberflächenempfangsspule (350, 550) eine Vielzahl von Spulenelementen (350, 352, 354, 550, 552) umfasst.

## Revendications

1. Dispositif (300, 500) pour soutenir une partie du corps, comprenant :
un manchon (310, 510) adapté pour supporter la partie du corps, le manchon contenant une chambre qui est configurée pour que sensiblement tout l'air contenu à l'intérieur soit évacué, **caractérisé par**
une bobine de réception de surface de radiofréquence flexible (350, 550) pour l'imagerie à résonance magnétique disposée dans la chambre.

2. Dispositif selon la revendication 1, dans lequel la partie du corps est un élément parmi un bras, un poignet, une épaule, une jambe, une cheville, une hanche, un bassin, un torse et un cou.

3. Dispositif selon la revendication 1, comprenant en outre :
une pluralité de particules disposées dans la chambre, dans lequel les particules comprennent des billes.

4. Dispositif selon la revendication 3, dans lequel les billes ont un diamètre sensiblement égal à deux millimètres.

5. Dispositif selon la revendication 3, dans lequel les billes comprennent du polystyrène.

6. Dispositif selon la revendication 1, comprenant en outre :
une sangle radiotransparente (330, 332, 334, 522, 524) pour fixer le manchon autour de la partie du corps.

7. Dispositif selon la revendication 6, dans lequel la sangle radiotransparente fixe par le biais de fixations à boucles et crochets.

8. Dispositif selon la revendication 1, comprenant en outre :
un orifice (380, 580) pour raccorder la bobine de réception de surface de radiofréquence flexible (350, 550) à un dispositif d'imagerie à résonance magnétique.

9. Dispositif selon la revendication 1, dans lequel la bobine de réception de surface de radiofréquence flexible (350, 550) comprend du cuivre.

10. Dispositif selon la revendication 1, dans lequel la bobine de réception de surface de radiofréquence flexible (350, 550) comprend une pluralité d'éléments de bobine (350, 352, 354, 550, 552).

11. Procédé comprenant :
la fixation d'un manchon (310, 510) autour d'une partie du corps blessée, le manchon (310, 510) contenant une chambre qui est configurée pour que sensiblement tout l'air qu'elle contient soit évacué, une bobine de surface de radiofréquence flexible (350, 550) pour que l'imagerie à résonance magnétique soit disposée dans la chambre ;
l'évacuation de sensiblement tout l'air de la chambre ;
le couplage de la bobine de surface de radiofréquence flexible (350, 550) à un dispositif à imagerie à résonance magnétique ; et
l'imagerie de la partie du corps blessée en utilisant le dispositif d'imagerie à résonance magnétique, la bobine de surface de radiofréquence flexible (350, 550) fonctionnant comme une bobine de réception pour l'imagerie.

12. Procédé selon la revendication 11, dans lequel la partie de corps blessée est un élément parmi un bras, un poignet, une épaule, une jambe, une cheville, une hanche, un bassin, un torse et un cou.

13. Procédé selon la revendication 11, dans lequel le manchon est fixé autour de la partie du corps blessée avec une sangle radiotransparente (330, 332, 334, 522, 524).

14. Procédé selon la revendication 11, comprenant en outre le couplage de la bobine de surface de radiofréquence flexible (350, 550) à un dispositif d'imagerie à résonance magnétique par le biais d'un orifice traversant le manchon (310, 510).

15. Procédé selon la revendication 11, dans lequel la bobine de réception de surface de radiofréquence flexible (350, 550) comprend une pluralité d'éléments de bobine (350, 352, 354, 550, 552).
